# EUROPEAN PATENT SPECIFICATION

(11) **EP 1 467 769 B1**
(45) Date of publication and mention of the grant of the patent: **28.05.2008**
(21) Application number: 02716134.8
(22) Date of filing: 18.01.2002
(51) Int. Cl.: A61K 47/48, A61K 31/465, A61K 9/20, A61K 9/68, A61P 25/34

(54) **USE OF A RESINATE IN THE PREPARATION OF A FORMULATION FOR SMOKING CESSATION**
VERWENDUNG EINES RESINATS ZUR HERSTELLUNG EINER FORMULIERUNG ZUR BEENDIGUNG DES RAUCHENS
UTILISATION D'UN RESINATE POUR LA PREPARATION D'UNE FORMULATION POUR ARRETER DE FUMER

(43) Date of publication of application: 20.10.2004
(73) Proprietor: Rohm and Haas Company, Philadelphia, PA 19106-2399 (US)
(72) Inventor: BELLAMY, Simon Andrew, c/o Rohm & Haas, Croydon, Surrey CR9 3NB (GB); HUGHES, Lyn, c/o Rohm & Haas, Croydon, Surrey CR9 3NB (GB); NICOLA, Mazin, Worthing, West Sussex BN11 4SD (GB)
(74) Representative: Kent, Venetia Katherine
(86) International application number: PCT/GB2002/000199
(87) International publication number: WO 2003/061709

(56) References cited:
- EP-A- 0 431 759
- EP-A- 0 911 039
- EP-A- 1 175 914
- EP-A- 1 175 915
- EP-A- 1 190 721
- WO-A1-00/40224
- GB-A- 1 101 366
- GB-A- 1 325 011
- US-A- 2 990 332
- US-A- 5 032 393
- IRWIN W J ET AL: "DRUG-DELIVERY BY ION-EXCHANCE. PART VII: RELEASE OF ACIDIC DRUGS FROM ANIONIC EXCHANGE RESINATE COMPLEXES" DRUG DEVELOPMENT AND INDUSTRIAL PHARMACY, NEW YORK, NY, US, vol. 16, no. 6, 1990, pages 883-898, XP001029685 ISSN: 0363-9045
- GARCIA-ENCINA G. ET AL: "In vivo evaluation of nylon-coated diclofenac-resin complexes" JOURNAL OF CONTROLLED RELEASE, ELSEVIER, AMSTERDAM, NL, vol. 23, no. 3, 1 March 1993 (1993-03-01), pages 201-207, XP000343200 ISSN: 0168-3659
- ANAND V. ET AL: 'Ion-exchange resins: carrying drug delivery forward' DDT - DRUG DISCOVERY TODAY vol. 6, no. 17, 17 September 2001, ELSEVIER SCIENCE LTD, GB, pages 905 - 914, XP001127363 ISSN: 1359-6446

## Description

### BACKGROUND OF THE INVENTION

It is well know in the art that using a complex formed between a polymeric material and an active substance can be beneficial. Such benefits can include changes in the release rate of drugs, taste masking of bitter drugs, control of the site of administration of drugs, control of the release of flavor substances, and stabilization of unstable substances.

The preparation of an active substance/ion exchange resin complex is called loading. The ion exchange resins complexed with the active substance are called resinates. The methods for loading have been varied, but in many cases are either problematic or limited in their application.

The typical method for loading active substances onto an ion exchange resin is to dissolve an acidic or basic, ionizable active substance in water, and then mix it with a suitable ion exchange resin. See, US2,990,332. The active substance is absorbed into the resin by the mechanism of ion exchange. The extent of loading will depend on several factors, including the rate of diffusion, the equilibrium constant, temperature, and the presence of other ions. The water is then removed by filtration, and the ion exchange resin dried by heating. As a general rule, anion exchange resins are useful for the loading of acidic substances, and cation exchange resins are useful for loading basic substances.

The need to dissolve the active substance to be loaded can lead to very large volumes of solution if the active substance has poor solubility in the loading medium. This leads to very low productivity in a commercial scale process. To overcome this problem, water miscible organic co-solvents such as ethanol are frequently used to increase the solubility and to reduce the total volume of solution. Introduction of these co-solvents into the process can add significant cost, because they are typically not recovered. They can increase the amount of hazardous waste generated, and introduce processing problems related to flammability and toxicity.

In the currently used commercial processes for making the resinates of active substances, said active substance is loaded onto a powdered, anion or cation ion exchange resin. The loading is performed in a predominantly aqueous system, whereby the active substance becomes immobilized on the resin by reaction with the functional groups of the resin. Use of an aqueous system for the loading has the disadvantage that the resulting slurry has to be dewatered and dried. This is currently achieved in a number of different ways, e.g., dewater in a decanter, and then dry in a vacuum dryer; or evaporate the water directly from the slurry in a vacuum distillation apparatus; and evaporate the water directly from the slurry using a spray dryer. There are problems associated with each of these methods. The decanter operation is made difficult because the ion exchange resin contains a significant fraction of very fine particles (<40 micron), and wet-cakes from such decanters can still contain >60% water by weight. The spray dryer and vacuum distillation operations are energy wasteful because all the water is removed by conversion to water vapor. Also, these methods can lead to particle agglomeration. Avoidance of these problems by using typical organic solvents leads to problems of toxicity from the residual solvent, safety problems from flammability, and environmental problems from vapor emissions and waste disposal.

The use of non-aqueous solvents as media for ion exchange reactions has been reported. See *"*Ion Exchange Resins" by Robert Kunin, p. 310, published by Robert E. Krieger Publishing Co, 1990. However, reaction times are reported to be very long for non-swelling solvents. Further, the solvents typically used are not optimum for industrial scale because they are flammable, or toxic, or difficult to remove efficiently, or difficult to reuse, or environmentally unacceptable, or high cost.

Many drug substances are hydrophobic and are poorly soluble in water. While this can be somewhat advantageous for absorption from solution into the gastrointestinal system, the actual dissolution of such drugs into physiological fluids can be very inefficient. This can result not only from a low solubility, but also a low rate of dissolution. This low rate of dissolution is itself the result of poor wettability of the hydrophobic solid, and the thermodynamic barrier caused by high crystal lattice energy which is difficult to overcome with water. This poor dissolution into physiological fluids can result in very poor and/or variable bioavailability of the drugs. Methods to improve the dissolution can thereby improve bioavailability.

For example, when nicotine is formulated into chewing gum and lozenges it is first loaded onto a cation exchange resin which has the effect of controlling the release rate of the nicotine during chewing or sucking in the mouth. Such complexes of nicotine with ion exchange resins are the subject of GB1325011. In agriculture it is used as a pesticide; and it is formulated as the nicotine sulfate salt in water, at a 40% concentration.

A number of solutions have been explored, including grinding the drug to very small particle size (WO99/30687) and supplying it as a solution in oils (EP0306236B1). Each of these techniques has disadvantages. For example, not all drugs can be ground to very fine particle size due to low melting point or heat sensitivity. Dissolution in oils or dispersion in other matrices severely restricts the formulation options. There is a need for a method to improve dissolution that does not suffer these disadvantages.

The use of ion exchange resins to improve the rate of dissolution of weakly ionic compounds was reported by Irwin. See, Irwin, et al, Drug Deliv. and Ind. Pharm, 16(6), 883 (1990). Irwin observed faster dissolution of mefenamic acid from a powdered, strong base anion exchange resin when compared to a solid suspension. The loading method used by Irwin employed an aqueous medium as known to those skilled in the art.

Thus, there is a need in the art for an active ingredient loading method that is environmentally friendly, safe, low cost, and capable of high productivity. There is also need for a method that improves the dissolution of poorly soluble drugs that is not limited by melting point or temperature sensitivity, and is compatible with most existing formulation methods. Applicants have surprisingly discovered a method that meets these needs and is applicable to nicotine.

The following terms have the following meanings herein:
The term "solubility," as used herein, means solubility as defined in the US Pharmacopoeia, 24, pg. 10. For the purposes of this invention the descriptor 'poorly soluble' will be used to describe substances that are very slightly soluble or practically insoluble in water by the USP definition. This solubility is <1 part of solute per 1000 parts of solvent. The descriptor 'soluble' will be used to describe substances with a solubility >1 part solute per 1000 parts solvent.

The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)

The term "resinate," as used herein, means an active substance/resin complex.

The terms "loaded" and "loading" as used here-in mean the preparation of a resinate. The amount of loading means the amount of active substance incorporated into the resin to form a resinate.
Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer (herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

Ion exchange resins are manufactured in different forms. These forms can include spherical and non-spherical particles with size in the range of 0.001mm to 2mm. The non-spherical particles are frequently manufactured by grinding of the spherical particles. Products made in this way typically have particle size in the range 0.001mm to 0.2mm. The spherical particles are frequently known in the art as 'Whole Bead.' The non-spherical particles are frequently known in the art as 'Powders.'

The present invention is as set out in the accompanying claims.

The present invention provides for the use of a resinate in the preparation of a formulation for smoking cessation, wherein the formulation comprises at least 0.25 wt % of the resinate, wherein the resinate comprises nicotine loaded onto a cation exchange resin, wherein the resinate is prepared by a method comprising steps of:
a. blending the nicotine with the resin and a solvent selected from the group consisting of a water miscible solvent selected from methanol, ethanol, isopropanol, n-propanol, acetone, dimethylformamide, tetrahydrofuran, dimethylsulfoxide, dimethylether, and acetic acid, tetrahydrofuran, dimethylsulfoxide, dimethylether, and acetic acid, a water-immiscible solvent or mixtures thereof to form an active substance/resin/solvent mixture;
b. maintaining said active substance/resin/solvent mixture, at a pressure and temperature that maintains said active substance resin/solvent mixture in the liquid state, for 1 second to 48 hours.

Preferably, said resins are suitable for human and animal ingestion.

Preferred cationic exchange resins include, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g, that are suitable for human and animal ingestion.

Most preferred cationic exchange resins include, styrenic weakly acidic cation exchange resin with a phenolic functionality with a weight capacity of 0.1 to 8.5 meq/g; and a styrenic strongly acidic cation exchange resin with a sulfonic acid functionality with weight capacity of 0.1 to 8 meq/g, or a methacrylic weakly acidic cation exchange resin with a carboxylic acid functionality with weight capacity of 0.1 to 12 meq/g.

Ion exchange resins useful in this invention have a moisture content between 0% and the water retention capacity of said resin.

Ion exchange resins useful in this invention are in powder or whole bead form.

Strongly acidic and weakly acidic cation exchange resins useful in the practice of the present invention are in the acid form or salt form or partial salt form.

The water soluble active substance is nicotine.

Nicotine useful in the practice of the present invention includes, that derived from the extraction of nicotine from the tobacco plant *Nicotiana tobacum.* It finds great use in the pharmaceutical and agricultural industries. In the pharmaceutical industry it is extensively used in smoking cessation formulations. In this use the nicotine can be administered in the form of lozenges, chewing gum, and inhalers.

The preferred nicotine useful in the practice of the current invention is nicotine that has an assay greater than 90% by weight.

The more preferred nicotine useful in the practice of the current invention is nicotine that has an assay greater than 95% by weight.

The most preferred nicotine useful in the practice of the current invention is nicotine that meets the purity specifications prescribed in the US PharmacopeiaUSP24, p1179.

Water miscible solvents useful in the practice of the present invention are methanol, ethanol, isopropanol, n-propanol, acetone, dimethylformamide, tetrahydrofuran, dimethyl sulfoxide, dimethyl ether, and acetic acid.

The preferred water miscible solvents are ethanol, isopropanol, n-propanol, and dimethyl ether.

The most preferred water miscible solvent is ethanol.

Water immiscible solvents useful in the practice of the present invention include, but are not limited to hydrocarbons, halogenated hydrocarbons, ethers, ketones, and esters having boiling points, at atmospheric pressure between 100°C and -100°C.

The preferred water immiscible solvents are fluorinated hydrocarbon solvents. In the method, a said fluorinated hydrocarbon solvent is preferably in the liquid state when in contact with the active substance.

A preferred fluorinated hydrocarbon is a C₁ to C₄ fluorinated hydrocarbon.

The C₁ to C₄ fluorinated hydrocarbon may be non-chlorinated. Preferably, it comprises one or more carbon, fluorine and hydrogen atoms only. Preferably, said fluorinated hydrocarbon is a C₁ to C₃, more preferably, a C₁ to C₂ fluorinated hydrocarbon. Especially preferred is a C₂ fluorinated hydrocarbon.

Said fluorinated hydrocarbon may include up to 10, preferably up to 8, more preferably up to 6, especially up to 4, fluorine atoms.

Said fluorinated hydrocarbon is preferably aliphatic. It is preferably saturated.

Said fluorinated hydrocarbon may have a boiling point at atmospheric pressure of less than 20°C, preferably less than 10°C, more preferably less than 0°C, especially less than -10°C. The boiling point may be greater than -90°C, preferably greater than -70°C, more preferably greater than -50°C.

Preferred water immiscible solvents are:
trifluoromethane (CF₃H);
fluoromethane (CH₃F);
difluoromethane (CF₂H₂);
1,1-difluoroethane (CF₂HCH₃);
1,1,1-trifluoroethane (CF₃CH₃);
1,1,1,2-tetrafluroethane (CF₃CFH₂)
pentafluoroethane (CF₃CF₂H);
1,1,1,2,2-pentafluorpropane (CF₃CF₂CH₃);
1,1,1,2,3-pentafluorpropane (CF₃CFHCFH₂);
1,1,1,2,2,3-hexafluoropropane (CF₃CF₂CFH₂);
1,1,1,2,3,3-hexafluoropropane (CF₃CFHCF₂H);
1,1,1,3,3,3-hexafluropropane (CF₃CH₂CF₃);
1,1,2,2,3,3-hexafluoropropane (CF₂HCF₂CF₂H);
1,1,1,2,2,3,3-heptafluoropropane (CF₃CF₂CF₂);
1,1,1,2,3,3,3-heptafluoropropane (CF₃CFHCF₃);

Tetrafluorethane is an especially preferred solvent with 1,1,1,2-tetrafluoroethane (CF₃CFH₂), also known as TFE, being most preferred. This solvent has a boiling point of -26.5°C at atmospheric pressure, is of low toxicity, is non-flammable, and is non ozone depleting.

Said solvent used in the prepration of a said resinate may include a fluorinated hydrocarbon solvent (especially TFE) as described together with one or more co-solvents. Said solvent may include less than 20wt%, preferably less than 15wt%, more preferably less than 10wt% of co-solvent.

A said co-solvent may be selected from: a C₂₋₆ hydrocarbon such as an alkane or cycloalkane with alkanes such as ethane, n-propane, i-propane, n-butane and i-butane being especially preferred; and hydrocarbon ethers, particularly dialkylethers such as dimethylether, methylethylether and diethyl ether. In other embodiments, said co-solvent may be polar, for example having a dielectric constant, at 20°C, of greater than 5. Such co-solvents may be selected from:
amides, especially N,N'-dialkylamides and alkylamides, with dimethylformamide and formamide being preferred; sulphozides, especially dialkyl sulphoxides, with dimethylsulphoxide being preferred; alcohols, especially aliphatic alcohols for example alkanols, with methanol, ethanol, 1-propanol and 2-propanol being preferred; ketones, especially aliphatic ketones, for example dialkyl ketones, with acetone being especially preferred; organic acids, especially carboxylic acids with formic acid and acetic acid being preferred; carboxylic acid derivatives, for example anhydrides, with acetic anhydride being preferred; cyanide derivatives, for example hydrogen cyanide and alkyl cyanides, with methyl cyanide and liquefied anhydrous hydrogen cyanide being preferred; ammonia; sulphur containing molecules including sulphur dioxide, hydrogen sulphide and carbon disulphide; inorganic acids for example hydrogen halides with liquefied anhydrous hydrogen fluoride, chloride, bromide and iodide being preferred; nitro derivatives, for example nitroalkanes and nitroaryl compounds, with nitromethane and nitrobenzene being especially preferred.

In a preferred embodiment when a fluorinated hydrocarbon solvent is used, substantially no co-solvent of the types described is used.

The preferred range of ratios of ion exchange resin to solvent is 1:1 to 1:1000, the more preferred range is 1:1.5 to 1:100, and the most preferred range is 1:2 to 1:5.

Preferably, the loading of nicotine in the resinate of the present invention is 1-100% of the ion exchange capacity of the resin, more preferably it is 5-100% of the ion exchange capacity of the resin, and most preferably it is 10-100% of the ion exchange capacity of the resin.

The preferred pressure range for the practice of the present invention is 5 to 35,000 kPascals, the more preferred range is 100 to 5000 kPascals, and the most preferred range is 350 to 700 kPascals.

The preferred temperature range for the practice of the present invention is -10°C to 100°C, the more preferred range is 0°C to 80°C, and the most preferred range is 5°C to 30°C.

The time to prepare a resinate of the present invention is from 1 second to 48 hours, preferably from 5 minutes to 12 hours, and most preferably from 5 minutes to 8 hours.

Preferably, a said method of preparing a resinate includes a step of separating a resinate comprising active substance/resin from solvent. This may suitably be done by filtration and/or vapourization of solvent. Thus, preferably the method includes the step of isolating the resinate, suitably in a solid form.

Said resinate is preferably formed into units comprising predetermined amounts of nicotine.

It may be difficult, in some cases, to remove all of the solvent used in the preparation of a resinate from the resinate itself and, accordingly, the resinate may be contaminated with a trace of solvent. Therefore, the invention extends a resinate comprising nicotine in combination with a resin, wherein the resinate includes a trace (e.g. less than 1wt%, preferably less than 0.5 wt%, especially less than 0.1wt% based on the total weight of resinate) of solvent.

Where a fluorinated hydrocarbon (e.g. tetrafluoroethane as described herein) is used in the preparation of a resinate, the invention extends to a resinate comprising an active substance in combination with a resin, wherein the resinate includes a trace (e.g. less than 1wt%, preferably less than 0.5wt%, especially less than 0.1 wt% based on the total weight of resinate) of a fluorinated hydrocarbon (e.g. 1,1,1,2-tetrafluoroethane).

The formulation includes at least 0.25 wt% of resinate. The formulation preferably includes 0.5 to 99 wt%, preferably 5 to 90wt% of resinate.

A said formulation includes said resinate in combination with another solid or liquid. The formulation may include at least 1 wt%, preferably at least 5wt%, more preferably at least 10wt%, especially at least 30wt% of said other solid or liquid.

Said solid or liquid may be a carrier, excepient or diluent.

Preferably, in a method of preparing a resinate as described herein, at least 0.5 kg, especially at least 1kg, of nicotine is blended with solvent. The nicotine and solvent may be blended for a time of less than 2000 hours preferably less than 1000 hours, more preferably less than 500 hours, especially less than 100 hours.

While reference Example 1 surprisingly illustrates that a poorly soluble drug can be loaded onto an ion exchange resin with less water than is required to completely dissolve said drug, the loading process takes about 2 hours and the mixture must be dewatered.

However, the addition of a water-immiscible or water miscible solvent as described hereinabove reduces the loading time to between 1 minute and 20 minutes, and eliminates the need to dewater the mixture. For example, in a preferred embodiment of the invention, the amount of water required is such that it does not exceed the water retention capacity of the ion exchange resin. In this way there is no separate water phase in the mixture. Because of the property of ion exchange resins to absorb water up to the water retention capacity the water can either be present in the ion exchange resin at the start of the process, or added as a separate ingredient to the mixture. The water immiscible solvent can be removed from the final mixture either by filtration, or by vaporization. The vaporization can be achieved by using heat, or by reducing the pressure, and providing a heat source to maintain the temperature of the solution between room temperature and the atmospheric pressure boiling point of said solvent. Specifically, the active substance, a suitable hydrated anion or cation exchange resin, and TFE are mixed at a pressure of about 520 kPascals to maintain said TFE in the liquid state. The mixture is stirred at room temperature for between 5 and 20 minutes. During this period the active substance rapidly loads onto the ion exchange resin, such that there is no solid active substance left in the mixture, and the amount of active substance dissolved in the TFE is insignificantly small. The TFE is then removed by reducing the pressure such that the TFE boils. The TFE vapor can be recovered either by using a condenser at less than the boiling point of the TFE, or by using a compressor and condenser. Both recovery methods are well known in the art. The TFE can then be re-used.

The following examples illustrate the practice of the present invention.

### REFERENCE EXAMPLE 1 - Water-Only Loading of a Poorly Water Soluble Active

Add 0.5 g of indomethacin, a poorly soluble active substance, and 1.5g of an acrylic anion exchange resin with tertiary amine functionality and a weight capacity between 5.8 and 6.2 meq/g, such as Amberlite IRA67, available from the Rohm and Haas Company, in its fully hydrated state to a 25 ml vial. Add 6 g of water to the mixture, close the vial and shake the mixture. After 2 hours the indomethacin will have disappeared and the ion exchange resin will be yellow. Drain the water from the mixture, to yield the wet resinate.

This experiment illustrates the very large reduction in required reaction volume achieved by the invention over the prior art. The solubility of indomethacin in water is 14ppm so that approximately 37kg of water would be required to completely dissolve the amount of indomethacin used in this example. For a commercial scale operation this decrease in required volume would represent a 6000 fold increase in productivity over the prior art.

### REFERENCE EXAMPLE 2 - Immiscible Solvent and Prehydrated Resin Loading of a Poorly Water Soluble Active

Proceed as in Example 1, except add 1.7g of water to the mixture. This is sufficient water to hydrate the ion exchange resin, but not sufficient to form a separate liquid water layer. After stirring for 10minutes stop the stirrer and allow the mixture to stand for a few minutes. It will be noted that the resin, now yellow in color, will float to the surface, and that there will be no indomethacin on the bottom of the vessel. Carefully remove approximately one half of the TFE as a liquid sample, without including any of the resinate. Remove the TFE from this sample by evaporation. It will be noted that there is no significant solid residue left after the TFE has been removed. These observations indicate that all the indomethacin loaded onto the resin.

### REFERENCE EXAMPLE 3 - Immiscible Solvent Loading of a Poorly Water Soluble Active

Proceed as in Example 1, except use 7g of dichloroethane. After shaking for 10 minutes, it will be noted that the resin is now yellow, and that there is no solid indomethacin present. This observation indicates the indomethacin loaded onto the ion exchange resin.

### PEFERENCE EXAMPLE 4 - Immiscible Solvent Loading of a Poorly Water Soluble Active

Proceed as in Example 1, except use 3.5g of pentane instead of dichloroethane. After shaking for 20 minutes, it will be noted that the resin is now yellow, and that there is no solid indomethacin present. This observation indicates the indomethacin loaded onto the ion exchange resin

### REFERENCE EXAMPLE 5 - Immiscible Solvent Loading of a Poorly Water Soluble Active

The same as Example 3, except use 1g of Nelfinivir, 1.4g of water, and 1.6g of a dried, ground methacrylic weakly acidic cation exchange resin with carboxylic acid functionality with weight capacity between 10.1 and 11.1 meq/g (such as Amberlite^{®} IRP64, available from the Rohm and Haas Company).

### REFERENCE EXAMPLE 6 - Immiscible Solvent and Prehydrated Resin Loading of a Poorly Water Soluble Active

In the same equipment as used in Example 2, charge 3g of an acrylic anion exchange resin with tertiary amine functionality and a weight capacity between 5.8 and 6.2 meq/g, such as Amberlite IRA67, available from the Rohm and Haas Company, in its fully hydrated state, whole bead form. To the same vessel charge 1g of indomethacin. Evacuate the air from the vessel, and then introduce 50g of 1,1,1,2-tetrafluoroethane (TFE) so that at the end of the addition the pressure is approximately 520 kPascals and the temperature is 20°C, such that the TFE is in the liquid state. Stir the mixture at room temperature for 10 minutes. During this period the resin will change to a yellow color, indicating indomethacin loading. Reduce the pressure in the loading vessel by venting it to the atmosphere to remove the TFE. There remains a water-wet resinate, that is indomethacin loaded onto the anion exchange resin.

### REFERENCE EXAMPLE 7 - Immiscible Solvent Only Loading Of A Poorly Water Soluble Active

Proceed as in Example 6, except dry the resinate in a vacuum oven at 60°C for 4 hours.

### REFERENCE EXAMPLE 8 - Miscible Solvent and Water (Due To Hydrated Resin) Loading Of A Poorly Water Soluble Active

Prepare a solution of 1g of indomethacin in 200ml of 50% aqueous ethanol. To this add 3g of an acrylic anion exchange resin with tertiary amine functionality and a weight capacity between. 5.8 and 6.2 meq/g (such as Amberlite IRA67, available from Rohm and Haas Company, Philadelphia, Pennsylvania) in its fully hydrated state, whole bead form. Shake the mixture overnight at room temperature. During this period the yellow solution will lose most of its color, and the resin will become yellow. Drain the solution from the mixture, and analyze it for indomethacin using a uv/vis spectrometer at a wavelength of 318nm, such as described in US Pharmacopoeia, USP24 p. 874. The analysis will indicate approximately 0.1g of the indomethacin was left in solution, and did not load onto the resin.

### REFERENCE EXAMPLE 9 - Miscible Solvent Loading of a Poorly Soluble Active

The same as Example 1 except that the instead of adding water, add 2.5g of water and 2.5g of ethanol. The indomethacin will load within 2 hours. The supernatant at the end of the experiment will contain approximately 0.003g of indomethacin that did not load.

### EXAMPLE 1-Immiscible Solvent Loading Of A Water Soluble Active

Construct equipment comprising a 150ml heavy walled glass vessel, capable of operating at more than 600 kPascals (the mix vessel) connected to a second identical vessel (the loading vessel) such that liquid in the mix vessel can be transferred into the loading vessel. Include valves and fittings in suitable places to allow complete evacuation of the system, charging of solvent (TFE) to the mix vessel, and the transfering of solvent from the mix vessel to the loading vessel. Charge 0.1g of nicotine with an assay greater than 95% to the mix vessel, and 10g of a dried, powder form, styrenic strongly acidic cation exchange resin with a sulfonic acid functionality with weight capacity of 4.3 to 4.9 meq/g(such as Amberlite^{®} IRP69 available from Rohm and Haas Company, Philadelphia, Pennsylvania) to the loading vessel. Evacuate the equipment to remove the air and then charge 50g of solvent (TFE) to the mix vessel. The pressure will rise to about 520 kPascals due to the vapor pressure of TFE. Stir the TFE and nicotine for 5 minutes to dissolve said nicotine and then transfer the solution to the loading vessel. Mix the slurry in the loading vessel for 18 hours and then reduce the pressure in the loading vessel by venting it to the atmosphere to remove the TFE. The remaining dry solid is nicotine loaded onto the cation exchange resin.

### EXAMPLE 2-Immiscible Solvent Loading Of A Water Soluble Active

Use commercial scale equipment that allows the same operations as described in Example 1, with the addition of a compressor connected to the receiver, a condenser attached to the outlet of the compressor, and a suitable pressure vessel for storage of TFE. Charge 18kg of nicotine that meets the purity requirements of the US Pharmacopeia 24, and charge 100kg of a methacrylic weakly acidic cation exchange resin with a carboxylic acid functionality with weight capacity of 10.1 to 11.1 meq/g (such as Amberlite^{®} IRP64 available from Rohm and Haas Company) to the loading vessel. Evacuate the equipment to remove the air. Seal the equipment to prevent entry of air, and then charge 360kg of TFE to the mixing vessel. Mix the nicotine and TFE for 15 minutes to dissolve the nicotine, and then transfer the solution to the loading vessel. Mix the slurry for at least 8 hours to allow the nicotine to be absorbed by the resin. Reduce the pressure in the loading vessel to 350 kPascals by operating the compressor. The TFE will distill into the compressor. Provide heat to maintain the loading vessel at 15°C. Operate the compressor to achieve >520 kPascals at the outlet, and operate the condenser to cool the compressed TFE to 15-20°C. When all the TFE has evaporated remove the resin loaded with nicotine (118 Kg) from the vessel.

## Claims

1. Use of a resinate in the preparation of a formulation for smoking cessation, wherein the formulation comprises at least 0.25 % by wt of the resinate, wherein the resinate comprises nicotine loaded onto a cation exchange resin, **characterized in that** the resinate is prepared by a method comprising steps of : a. blending the nicotine with the resin and a solvent selected from the group consisting of a water miscible solvent selected from methanol, ethanol, isopropanol, n-propanol, acetone, dimethylformamide, tetrahydrofuran, dimethyl sulfoxide, dimethylether, and acetic acid, a water-immiscible solvent or mixtures thereof to form an active substance/resin/solvent mixture; b. maintaining said active substance/resin/solvent mixture, at a pressure and temperature that maintains said mixture in the liquid state, for 1 second to 48 hours.

2. A use according to Claim 1, wherein the solvent is a water immiscible solvent.

3. A use according to Claim 2, where in the nicotine is loaded at 100% of the ion exchange capacity of the resin.

4. A use according to claim 1, wherein said solvent comprises a C₁ to C₄ fluorinated hydrocarbon.

5. A use according to claim 4, wherein said fluorinated hydrocarbon includes carbon, fluorine and hydrogen atoms only.

6. A use according to claim 4, wherein said fluorinated hydrocarbon is tetrafluoroethane.

## Patentansprüche

1. Verwendung eines Resinats zur Herstellung einer Formulierung zur Beendigung des Rauchens, wobei die Formulierung mindestens 0,25 Gewichtsprozent des Resinats umfasst, wobei das Resinat auf ein Kationenaustauscherharz geladenenes Nikotin umfasst, **dadurch gekennzeichnet, dass** das Resinat durch ein Verfahren hergestellt ist, umfassend die Schritte
a. des Mischens des Nikotins mit dem Harz und einem Lösungsmittel, ausgewählt aus der Gruppe, bestehend aus
einem mit Wasser mischbaren Lösungsmittel, ausgewählt aus Methanol, Ethanol, Isopropanol, n-Propanol, Aceton, Dimethylformamid, Tetrahydrofuran, Dimethylsulfoxid, Dimethylether, und Essigsäure,
einem mit Wasser nicht mischbaren Lösungsmittel, oder Gemischen davon, um ein aktive Substanz/Harz/Lösungsmittel-Gemisch zu bilden,
b. des Beibehaltens des aktive Substanz/Harz/Lösungsmittel-Gemisches für eine Sekunde bis 48 Stunden bei einem Druck und einer Temperatur, welche das Gemisch im flüssigen Zustand halten.

2. Verwendung nach Anspruch 1, wobei das Lösungsmittel ein mit Wasser nicht mischbares Lösungsmittel ist.

3. Verwendung nach Anspruch 2, wobei das Nikotin zu 100% der lonenaustauschkapazität des Harzes beladen ist.

4. Verwendung nach Anspruch 1, wobei das Lösungsmittel ein C₁ bis C₄ fluorierter Kohlenwasserstoff ist.

5. Verwendung nach Anspruch 4, wobei der fluorierte Kohlenwasserstoff nur Kohlenstoff-, Fluor- und Wasserstoffatome einschließt.

6. Verwendung nach Anspruch 4, wobei der fluorierte Kohlenwasserstoff Tetrafluorethan ist.

## Revendications

1. Utilisation d'un résinate dans la préparation d'une formulation pour arrêter de fumer où la formulation comprend au moins 0,25 % en masse de résinate, ou le résinate comprend de la nicotine chargée sur une résine échangeuse de cations, **caractérisée en ce que** le résinate est préparé par un procédé comprenant les étapes de : a. mélange de la nicotine avec la résine et un solvant choisi dans le groupe consistant en un solvant miscible à l'eau choisi parmi le méthanol, l'éthanol, l'isopropanol, le 1-propanol, l'acétone, le diméthylformamide, le tétrahydrofurane, le diméthylsulfoxyde, le diméthyléther et l'acide acétique, un solvant immiscible à l'eau ou des mélanges de ceux-ci pour former un mélange substance active/résine/solvant ; b. maintien dudit mélange substance active/résine/solvant à une pression et une température qui maintiennent ledit mélange à l'état liquide, pendant 1 seconde à 48 h.

2. Utilisation selon la revendication 1, où le solvant est un solvant immiscible à l'eau.

3. Utilisation selon la revendication 2, où la nicotine est chargée à 100 % de la capacité d'échange d'ions de la résine.

4. Utilisation selon la revendication 1, où ledit solvant comprend un C₁-C₄ hydrocarbure fluoré.

5. Utilisation selon la revendication 4, où ledit hydrocarbure fluoré inclut des atomes de carbone, de fluor et d'hydrogène seulement.

6. Utilisation selon la revendication 4, où ledit hydrocarbure fluoré est le tétrafluoroéthane.
